(19) **Europäisches Patentamt**
**European Patent Office**
**Office européen des brevets**

(11) **EP 2 284 493 A1**

(12) **EUROPEAN PATENT APPLICATION**

(43) Date of publication:
**16.02.2011 Bulletin 2011/07**

(51) Int Cl.:
***G01C 22/00*** (2006.01)   ***A61B 5/103*** (2006.01)
***A63B 69/00*** (2006.01)   ***G01P 1/12*** (2006.01)

(21) Application number: **09010312.8**

(22) Date of filing: **10.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Cairos technologies AG**
**76307 Karlsbad (DE)**

(72) Inventors:
- **Habel, Thorsten**
  **75045 Walzbachtal (DE)**

- **Gierich, Martin**
  **76297 Stutensee (DE)**
- **Fendler, Matthias**
  **76307 Karlsbad (DE)**

(74) Representative: **Grünecker, Kinkeldey, Stockmair & Schwanhäusser**
**Anwaltssozietät**
**Leopoldstrasse 4**
**80802 München (DE)**

(54) **Sensor-based tracking of variable locomotion**

(57)    The invention relates to a method, device and system for the sensor-based tracking of a variable locomotion, and, in particular, to a method, device and system for acceleration-sensor based measurement of the highly erratic movement of a team sports player. The method comprises continuously measuring acceleration values corresponding to the locomotion of a person. For a single step of the person, a type of locomotion is determined by analyzing a temporal sequence of acceleration values of a portion of the acceleration values corresponding to the single step. Further, a swing time and at least one characterizing acceleration value are determined from the acceleration values for the single step and the step length is determined based on the type of locomotion, the swing time and the at least one characterizing acceleration value.

FIG. 4

**Description**

The invention relates to a method, device and system for the sensor-based tracking of variable locomotion and, in particular, to a method, device and system for acceleration sensor-based measurement of the highly erratic movement of a team sports player.

There is increasing interest in providing professional, semi-professional and non-professional athletes and sportsmen with means for accurately measuring their total distance covered on a track or playing field and for measuring, recording and visualizing a profile of their velocity, step lengths and ball possession periods.

Known solutions, comprising pedometers and simple running sensors, are specifically adapted to the needs of runners and work with sufficient accuracy when evaluating jogging or sprint periods of a specific mean velocity or step length. The employed devices are particularly suitable for being used by a runner abiding by a particular gait, step frequency and step length and thus a quite narrow velocity range.

These known solutions, however, lead to problems in more erratic environments or when extending the application field to more diverse types of locomotion, including, for example, locomotion in team sports or nordic walking, competitive walking, cross-country skiing, downhill sports, roller skiing, roller skating, skating, swimming, rowing, paddling or the like, where cyclic motion is present, but the assumptions necessary for the simplified pedometer or simple running sensors do not hold true anymore. In team sports, for instance, one single person frequently switches between different types of locomotion and gait, including for example forward motion; comprising walking, jumping, jogging and sprinting; backward motion; sideways motion; jumping or jogging on the spot and combinations of the aforementioned with touch of ball events.

Pedometers attempt to measure both distance and velocity essentially by stride counting, meaning that distance and speed can only be estimated if a stride length is known and stride length consistency may be assumed. But even for rather homogeneous locomotion activities, an individual's stride length can change considerably from day to day or even within one session due to changes in terrain, exhaustion, interval training or other factors. Therefore, a calibration by running a known distance is not sufficient to widen the application range of such pedometers.

US 6,513,381 B2 proposes a different method and system based on determining a net horizontal acceleration, which may be integrated to find a subject's gait speed and again integrated to calculate the distance covered by the subject. For this purpose, at least one pair of accelerometers and a tilt sensor mounted in fixed relation to a reference plane of a shoe, preferably the shoe's sole, are used for extracting kinematic variables, including linear and rotational acceleration. However, double integrations for determining position information involve various problems that may negatively effect the accuracy of this method. First, the gravitational acceleration is not easily excluded from the calculations without knowing absolute tilt degree values. Second, sensor errors or inaccuracies propagate from the initial formulas through to end results achieved by the double integrations. Most importantly, integration always leads to problems when it comes to accurately determining the unknown integration constants. Finally, numerical integration is very costly in terms of processing power and processing speed, and additionally induces further inaccuracies. Moreover, the described motion analysis system focuses on forward motion and does not provide for an initial distinction with regard to the type of locomotion of the subject of the analysis, thus applying the same complex double integration algorithm regardless of the subject's actual motion.

CA 261 52 11 A1 relates to a significantly reduced complexity solution using one acceleration sensor without any inclination compensation. In particular, characteristic accelerations are determined for a step cycle, which are measured by means of the acceleration sensor and allow for calculating an approximate velocity by applying variable parameters to the characterizing acceleration. After further obtaining a time value used up in one pair of steps, a step length is calculated from the determined velocity and said time value. Different forms of motion may be distinguished based on the value of the maximum, minimum, or characteristic accelerations and/or the step rate.

However, the previous solutions described above are either not applicable to highly erratic locomotion patterns or are too costly in terms of processing power and speed, while still not providing a satisfying accuracy, and are furthermore restricted to inflexible calculation schemes.

It is therefore the object of the present invention to provide an efficient method, device and system that affords the accurate measurement of at least the covered distance and velocity of a team sports player.

This object is solved by the subject matter of the independent claims.

Preferred embodiments are defined in the dependent claims.

A method of measuring a person's movement implemented by one or more portable devices is provided. The method comprises the continuous measurement of acceleration values associated with the locomotion of a subject, i.e. a person. For a single step of the person, the type of locomotion is determined by analyzing a temporal sequence of acceleration values of a portion of the acceleration values corresponding to the respective step. Additionally, a swing time and at least one characterizing acceleration value is determined for the step from the acceleration values, and the step length is determined based on the type of locomotion previously determined, the swing time and the at least one characterizing acceleration value.

According to an aspect of the present invention, a velocity is calculated which is associated with the locomotion of the person based on the determined step length.

According to another aspect of the present invention, for each portion of the acceleration values measured a step length is determined and the distance covered by the person on the underlying surface is calculated based on the determined step length.

According to yet another aspect of the present invention, a plurality of parameter sets and/or calculation formulas is stored, wherein each parameter set is assigned to a specific type of locomotion. Determining a step length comprises applying a set of parameters assigned to the determined type of locomotion.

According to another aspect of the present invention, each of the parameter sets may further correspond to at least one velocity range; a characteristic of acceleration values corresponding to a sequence of steps; and data corresponding to a person, comprising physical properties of the person, including age, weight and fitness level, and/or individually input selection parameters. A specific parameter set may be individually calibrated by comparing step lengths, derived distances covered by the person or velocity profiles, which all are determined based on the parameter set without further calibration, with step lengths, distances or velocities determined with a high-precision optical tracking system. The results of the comparison are subsequently applied to the parameter set to modify the parameter set according to this further calibration.

According to an aspect of the present invention, the measured acceleration values are multi-dimensional acceleration values sensed by at least one acceleration sensor for up to three-dimensional acceleration measurement. According to an aspect, only the temporal sequence of a specific one-dimensional preferential component of the multi-dimensional acceleration values is analyzed for the temporal sequence of acceleration values, while characterizing acceleration values are determined for at least two one-dimensional components of the multi-dimensional acceleration. Preferably, the preferential one-dimensional component is comprised in these one-dimensional components. The characterizing acceleration values are preferably determined for each step based on a maximum, minimum, mean or weighted mean, or other derived value corresponding to the underlying portion of acceleration values.

According to another aspect of the present invention, the acceleration values are measured with at least one acceleration sensor attached to one or more limbs of the athlete.

According to the invention, a measuring device is provided for measuring movement of a person. The device comprises at least one acceleration sensor for continuously measuring acceleration values associated with the locomotion of a person in at least two dimensions. According to an aspect, the at least one acceleration sensor is a multi-dimensional acceleration sensor for measuring three-dimensional acceleration values. The measuring device further comprises storing means for storing a plurality of parameter sets, wherein each parameter set is assigned to a specific type of locomotion. The device further comprises a processing means for assigning a type of locomotion to a step of the person by analyzing a temporal sequence of acceleration values of a portion of the acceleration values corresponding to the step; determining, for the step, a swing time and at least one characterizing acceleration value from the acceleration values; and determining a step length based on the swing time, the at least one characterizing acceleration value and the type of locomotion.

According to an aspect of the present invention, the processing means is further adapted to calculate the velocity associated with the locomotion of the person from the step length and/or to determine a step length for each portion of the acceleration values; and/or to calculate the distance covered by the person based on the determined step lengths.

According to another aspect, the measuring device further comprises a communication means for exchanging data with at least one of a receiver device, a game ball, a display means and a computing device.

According to yet another aspect of the present invention, the measuring device is a foot pod adapted for attachment to a sports shoe or insertion into the sole of a sports shoe.

A system is provided comprising the measuring device and a receiver device for receiving data from the measuring device, storing the data, displaying a graphical representation of the data and/or transmitting sets of the stored data to an external computing device. The system may further comprise a game ball adapted to send touch detection signals to the measuring device.

The present invention is based on the notion that a type of locomotion, including typical types of locomotion performed in team sports such as soccer, handball, basketball, football and the like, but also types of locomotion employed in other cyclic locomotion activities, may be distinguished by analyzing a temporal sequence of measured acceleration values. According to the invention, this insight is employed in determining a specific type of locomotion corresponding to a step in the cyclic motion of a person, and a step length is calculated on the basis of the knowledge of the type of locomotion. According to aspects of the present invention, different individualized and specifically calibrated calculation formulas and parameter sets may be employed based on the determined type of locomotion for each single step of the person.

Accordingly, it is an advantage of the present invention to produce precise measurement results for distance, velocity and step lengths in a wide field of applications with a particular suitability for team sports where the kind of movement employed by a player frequently changes and prior art solutions are insufficient as they are only applicable to homogeneous cyclic motions within a predetermined, small velocity window.

A further advantageous aspect of the present invention is that an analysis of a temporal sequence of acceleration values of a portion of the measured acceleration values can be performed in a very efficient and simple manner by, for example, employing a state machine or similar concepts that concentrate on the succession of particular states or events in a measured acceleration, comprising, for instance, upward or downward zero crossings and step cycle specific absolute or local maxima and minima.

BRIEF DESCRIPTION OF THE DRAWINGS

The above and other aspects, features and advantages of the embodiments of the present invention will become more apparent in the following detailed description when taken into consideration in conjunction with the accompanying drawings, in which:

Figure 1 is a block diagram illustrating an exemplary system according to an embodiment of the present invention;

Figure 2 is a block diagram illustrating an exemplary device for measuring movement of a person according to an embodiment of the present invention;

Figure 3 is a schematic drawing illustrating implementation of acceleration sensors and/or measuring devices according to an embodiment of the present invention;

Figure 4 is a flow chart illustrating a method according to embodiments of the present invention;

Figures 5A, 5B and 5C show charts illustrating measured accelerations and derived values according to embodiments of the present invention for forward motion, backward motion and mixed motion, including touch of ball events;

Figures 6A and 6B show charts illustrating relative errors in step length determination according to embodiments of the present invention;

Figures 7A and 7B show charts illustrating speed profiles determined according to an embodiment of the present invention in comparison with speed profiles according to an optical tracking system; and

Figure 8 shows a schematic drawing illustrating placement of a foot pod according to embodiments of the present invention.

DETAILED DESCRIPTION OF EXEMPLARY EMBODIMENTS

Exemplary embodiments of the present invention will now be described in detail with reference to the annexed drawings.

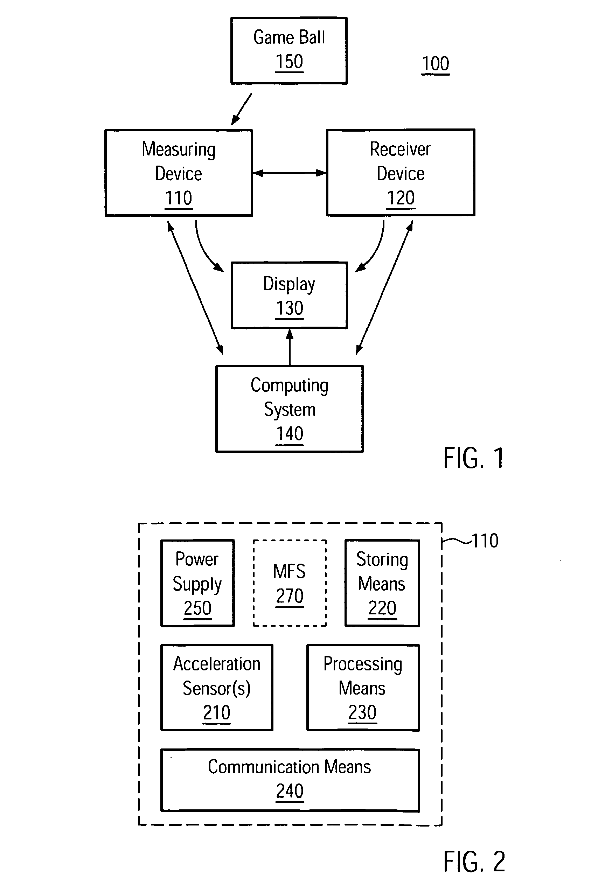

Referring to Figure 1, the system 100 comprises a measuring device 110 for measuring movement of a person and, in particular, quantities related to the locomotion of a team sport player according to embodiments described below.

The measuring device is attached to a limb of the player and in some embodiments to a shoe of the team sport player. Alternatively, the measuring device 110 is attached to a wrist or the hip of the player. The system further comprises a receiver device 120, which may be separate from or integrated into the measuring device 110. If the receiver device is distinct from the measuring device, the measuring device 110 communicates measured data to the receiver device 120 by means of radio communication or other suitable short to mid-range communication signals. The system 100 further comprises a display means 130, which is separate from or, alternatively, integrated into the measuring device 110 or the receiver device 120. The display means 130 is adapted to display graphical representations of measured or derived data received from the measuring device 110, the receiver device 120 or a computing system 140, which is in communication with the measuring device 110 and/or the receiver device 120. Alternatively, according to an embodiment, the measuring device 110 directly stores measured and derived data and can optionally include a display means 130 to display graphical representations or visualizations of the measured and derived data. Alternatively, measured and/or derived data and quantities are communicated to the receiver device 120, which stores and displays the quantities associated with one or more training or playing sessions of the team sport player. Either the measuring device 110 or the receiver device 120 are selectively in communication with an external computing system 140, which comprehensively evaluates and visualizes the collected data by means of an internal or external display means 130. According to an embodiment, the computing system and/or the receiver device 120 are media players capable of communicating with the measuring device 110 or the receiver device 120. An application program may be employed to upload collected data to a website or distributed database for comprehensive visualization of and comparison with a multitude of other team sport players.

Referring now to Figure 2, the measuring device 110 includes an active or passive power supply means 250. The system optionally further comprises a magnetic field sensor 270 for measuring the earth magnetic field or a magnetic field generated by a magnetic field generator included in a game ball or goal posts. Measuring the earth magnetic field according to an embodiment facilitates determination of a stance phase, during which the inclination relative to the earth magnetic field is constant and reoccurs with each cyclically repeating stance phase.

Measuring device 110 further comprises a storing means 220. According to an embodiment, a multitude of parameter sets and calculation instructions and formulas are stored in storing means 220. Each of the parameter sets and calculation instructions and formulas is assigned to one specific type of locomotion. According to an exemplary embodiment of the present invention, a step length is calculated in accordance with a calculation formula employing the square of a determined time for a single swing motion, while, according to an alternative embodiment, a step length is linearly dependent on a determined swing phase time. Moreover, according to the determined type of locomotion, one, two, three or more characterizing acceleration values may be employed in calculating a step length. In general, for arbitrary types of locomotion, each acceleration component of a three-dimensional acceleration measured by one or more acceleration sensors is utilized. For instance, in an embodiment where the measuring device 110 is inserted in the sole of a sports shoe and the analyzed team sports player is a soccer player, even a seemingly simple forward motion of the soccer player may involve arbitrary rotations of the foot and thus the sports shoe, thereby involving accelerations in each of the y, x and z directions. In the following, the y-direction shall denote the preferential direction of the measuring device 110, which in an exemplary placement of the measuring device 110, for instance in a sports shoe adapted for such insertion, coincides with the forward direction of the sports shoe within a reference plane of the sports shoe, for instance defined by the sole of the shoe in rest. According to this activation of each of the three-dimensional components of acceleration, one or more characterizing values for $a_y$, $a_x$ and $a_z$ are applied in calculating an accurate step length.

Measuring device 110 comprises one or more one-dimensional or multi-dimensional acceleration sensors 210. According to exemplary embodiments of the invention, acceleration sensors optimized for an acceleration range of $\pm 8g$ are employed. Similar acceleration sensors are used in mobile terminals for display orientation determination or in navigation devices.

Measuring device 110 further comprises processing means 230 for analyzing the measured acceleration values, analyzing in particular a temporal sequence of acceleration values of portions of the measured acceleration values and determining a type of locomotion on the basis thereof. Processing means 230, on the basis of the determined type of locomotion, a swing time and one or more characterizing acceleration values determined using the acceleration values, determines a step length, a velocity and a total distance covered derived therefrom. According to an embodiment, the analysis of a temporal sequence of acceleration values or of characterizing events in an acceleration curve derived from the acceleration values is performed for only one component of the acceleration values. Preferably, this component is the acceleration component corresponding to the preferential direction denoted previously with the y-axis. According to alternative embodiments, to achieve a sufficiently precise evaluation of a lateral movement, the temporal sequence of at least another one-dimensional component of the acceleration is analyzed in determining the type of locomotion.

Measuring device 110 further comprises communication means 240 for communicating raw or processed data and stored data collections to a receiver device 120, a computing system 140 or a display 130. Communication means 240 also receives instructions or an upload of parameter sets from receiver device 120 or an external computing system 140. Furthermore, according to an embodiment, communication means 240 receives touch of ball detection signals generated by a device in a game ball. The comparison of a temporal coincidence of a characteristic in the measured acceleration values with the receipt of such a touch detection signal received from a game ball allows validation of a contact event, a respective determination of the type of locomotion including a ball contact event, and a subsequent storage of the contact event in storage means 220 or the communication of the validated contact event to receiver device 120 or external computing system 140.

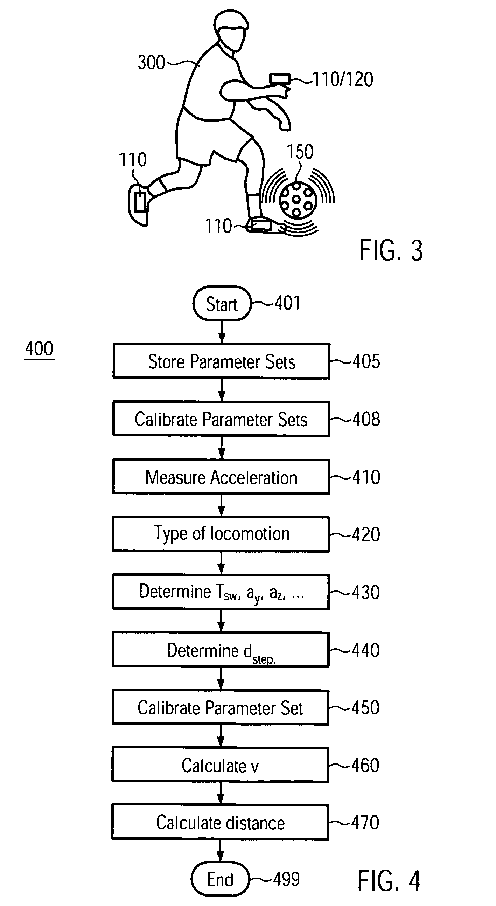

Referring now to Figure 3, a professional, semi-professional or non-professional sportsman or team sport player equipped with one or more devices according to the present invention is schematically illustrated. The measuring device 110, with or without integrated receiver device 120, may be attached to a limb 310, ..., 340 of the person 300 by various means and in various forms. According to a preferred embodiment of the invention, a measuring device 110 is attached to each foot of the person. Preferably, this is accomplished by attaching the measuring device 110 to or inserting it into a sports shoe. The redundant provision of two measuring devices 110 increases the accuracy of measured quantities by allowing a respective averaging and/or error detection. Moreover, redundant provision of measuring devices 110 provides for recognizing every ball contact event corresponding to tackles, passes and shots performed with both feet of the sportsperson and thus provides a more precise step length determination and more comprehensive collection of data related to the sport session performed by the person at the expense of increasing total costs.

If game ball 150 recognizes a ball contact event, which may be accomplished by respective pressure sensor arrangements or acceleration sensors comprised in the game ball 150, it undirectedly communicates the recognized ball contact event to any devices located in the vicinity of the game ball 150. According to an embodiment of the invention, a receiving device, which may be measuring device 110, compares the time of receipt of such a ball contact detection signal with characteristics obtained from the measured acceleration values that indicate the possible occurrence of a ball contact. In case of a coincidence, the measuring device 110 registers a verified ball contact event and utilizes the information that a ball contact has occurred during a step cycle corresponding to measured acceleration values in determining the accurate type of locomotion and a corresponding parameter set and calculation instructions for determining the step length.

According to alternative embodiments, the measuring device 110, for instance employed by a swimmer or runner, may be integrated into a wrist device including a wristwatch or a wristband. Such applications including acceleration measurements of the torso, an arm or a hand require different calibrations and calculation instructions than those applicable to measuring acceleration at the foot level, but are also embraced in the scope of the present invention.

Alternatively, only the receiver device 120 may be integrated into a wristwatch or a hip or torso belt attached to person 300.

Referring to Figure 4, a flow chart is shown illustrating a portable device-implemented method of measuring movement of a person according to embodiments of the present invention. The method starts at step 401. A multitude of parameter sets and calculation instructions, wherein each combination of parameter set and calculation instructions is at least assigned to a specific type of locomotion, are stored in a portable measuring device 110 or a combined device comprising a measuring device 110 and a receiver device 120, step 405. Alternatively, the parameter sets and calculation instructions are stored on a receiver device 120, which is distinct from the measuring device 110. According to this embodiment, measuring device 110 communicates the measured acceleration values in raw or processed format to receiver device 120 for further processing.

At step 408, one or more of the parameter sets are calibrated for specific velocity ranges or various characteristics of typical types of locomotion, including for instance a case of locomotion where the swing phase is approximately constant across different kinds of gaits employed by the athlete in a forward motion, including walking, jogging and sprinting. Moreover, a parameter set can be calibrated in step 408 corresponding to data individually applicable to the subject of the measurement, comprising physical properties of the person including age, weight and fitness level, other individually input selection parameters, or parameters indicating a specific type of activity including a type of team sport or swimming, cross-country skiing or the like. In step 410, acceleration values according to the locomotion of the subject are continuously measured.

In step 420, for a single step or movement cycle of the person, a type of locomotion is determined by analyzing a temporal sequence of acceleration values of a portion of the acceleration values corresponding to the step or cycle in the measured acceleration value pattern. According to an embodiment, the temporal sequence is determined by employing a state machine, which only registers state transitions according to the measured acceleration or by extrapolating or interpolating the measured acceleration values to determine when and in which sequence maximum or minimum values of acceleration and upward and downward zero crossings of the acceleration component(s) under analysis occur. For the step or cycle for which a step length is to be determined, a time value corresponding to the cyclic movement, swing time $t_{SW}$, and, according to embodiments, at least one characterizing acceleration value including *ay*, *ax* and *az* or more than one characterizing acceleration value, for instance for each one-dimensional component of the measured acceleration values, is determined in step 430. Based on the determined type of locomotion, the time value corresponding to the swing time and the at least one characterizing acceleration value, the step length corresponding to the step or the movement cycle is determined in step 440.

According to an embodiment, it is possible to select a post-measurement or a measurement-related calibration, step 450. Calibration 450 comprises calculating or modifying a parameter set by comparing determined step lengths, a derived total distance covered or derived velocities, wherein all of these quantities are determined based on an initial parameter set selected from the stored parameter sets according to the determined type of locomotion, with either known step lengths, total distances covered or velocities, or step lengths, distances or velocities measured with a high-precision optical tracking system. The results of the comparison are subsequently applied to the parameter set, thereby modifying the individual parameter values comprised in the parameter set.

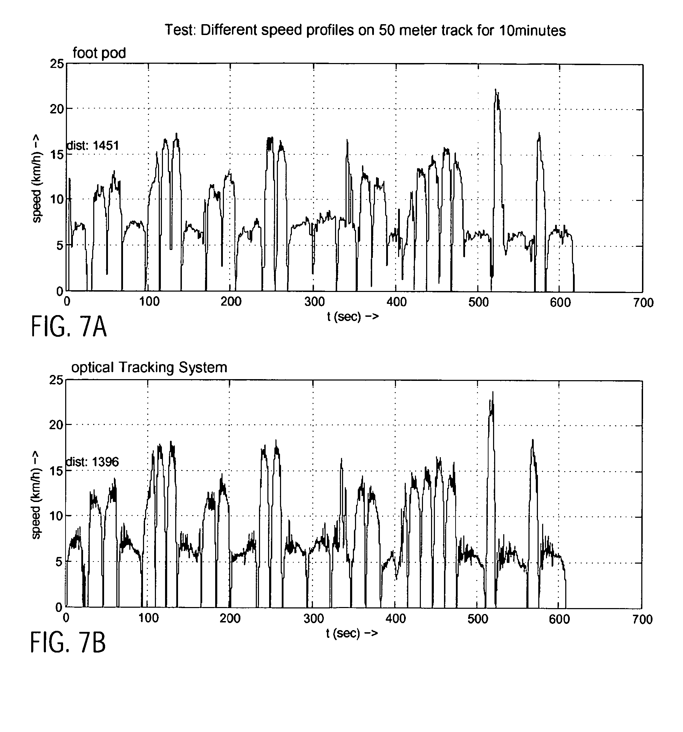

Figures 7A and 7B show example charts illustrating a comparison, in this case for velocities determined according to embodiments of the present invention and velocities measured for the same person and movement pattern by a high-precision optical tracking system. More precisely, the charts show speed profiles for a multitude of 50m track runs measured with a system and method according to embodiments of the present invention (Fig. 7A) and corresponding speed profiles measured with the high-precision optical tracking system (Fig. 7B). The shown results correspond to already pre-calibrated parameter sets according to calibrations as described above. Similar comparisons are performed in the process of the calibration of a raw, initial parameter set to generate a more accurate parameter set achieving a higher accuracy in generating quantities according to the present invention.

In general, according to the present invention, the measuring device 110 is calibrated with an optical tracking system as described above. High-precision optical tracking systems are suitable for determining highly accurate total distances, paths taken on the playing field and velocities. These accurate measurements are utilized in calibrating the parameters used in determining quantities according to an embodiment of the invention.

Equation (1) shows an exemplary general calculation formula for the step length:

$$(1)\quad d_{step} = C1 \cdot ay \cdot t + C2 \cdot 2az \cdot t + C3 \cdot ax \cdot t^2 + C4 \cdot ay \cdot t^2 + C5 \cdot az \cdot t^2$$

The mean velocity determined for the swing phase of the foot by such a high-precision optical tracking system can be used in initially determining calibration parameters c1...c5 and in later modifying and refining the calibration parameters to determine more appropriate calibration parameters C1... C5 utilized in determining a more precise step length. According to an embodiment, the whole velocity range typically covered by a team sports player can be calibrated in this manner by subdividing the entire velocity range into suitable velocity subranges and calibrating a parameter set for each subrange.

In step 460, a velocity is calculated based on the determined step length. According to an embodiment, a first calculated velocity is a foot swing phase or, more generally, a limb swing phase velocity calculated from a step or cycle length and a determined swing time represented by a dividing of the step length with the swing time, $d_{step}$ / $t_{SW}$. Furthermore, a velocity of movement projected on the surface traversed by the person is calculated based on the step length. To calculate this latter velocity, a cycle or step time is determined from the acceleration values by determining equivalent points in the cyclic acceleration pattern and measuring a corresponding time period separating the two equivalent points.

To illustrate the relation between a determined step length, a velocity of the person and a swing velocity, it is advantageous to consider the different phases traversed during a typical gait cycle. A swing phase, according to biomechanical science terminology, is subdivided into an early swing and a late swing. The early swing starts with an acceleration phase, continues with a mid-swing phase and a transition phase and transitions into the late swing phase with a deceleration. The stand or support phase of the foot, also called the contact phase, is subdivided into a loading phase, a mid-stance phase and a drive-off phase. The phases during the contact phase are traversed in a physiological sequence of heel strike, foot flat, mid-stance and toe-off. The described phases relate to a typical walking gait, where the stance phase represents 60 percent of the total cycle time period and the swing phase represents 40 percent. In typical running, the stance phase is reduced to 40 percent of the whole cycle time period, while the swing phase increases to a 60 percent portion. Accordingly, the above-mentioned scenario of a constant swing phase time is possible even though a step frequency will typically be higher while jogging or sprinting than during walking.

Returning to the calculation of the foot swing velocity or a velocity of the person on the playing field or track, the foot swing velocity according to the invention is calculated based on the swing phase time, while the velocity of the person on the playing field or track, i.e. the horizontal velocity of the person, is calculated based on the total time period of a gait cycle or locomotion cycle. At step 470, a total distance covered by the person is calculated by determining a step length for each portion of the measured acceleration values during a time period of interest and performing a sum over the step lengths determined by measuring device 110 for a single foot. According to an alternative embodiment, the step lengths determined for each foot are averaged and the averaged step lengths are summed up to calculate the distance covered by the person. The method ends at step 499.

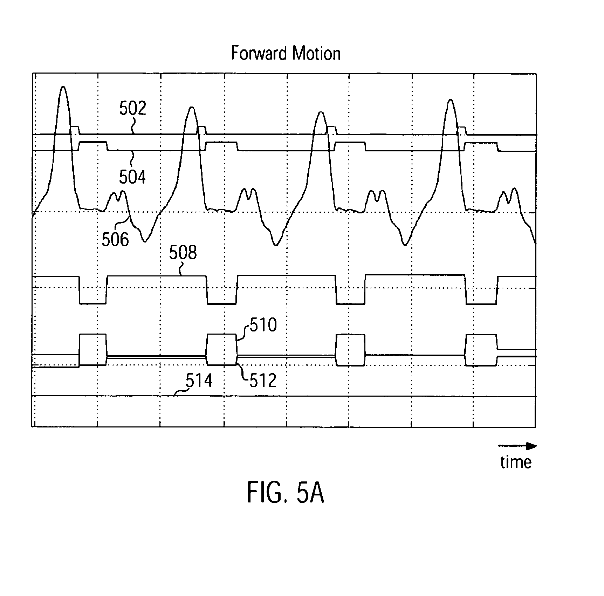

According to a preferred embodiment, a state machine is employed in step 420 for analyzing a temporal sequence of acceleration values. Figures 5A to 5C show charts illustrating a curve derived from measured acceleration values, derived signals and determined step lengths according to an embodiment of the present invention.

Referring now to Figure 5A, the shown signals and curves correspond to a forward motion. Curve 506 represents the one-dimensional component *ay* of the measured acceleration values, which is the acceleration component measured for the direction of preference, which for instance is the forward direction of the measuring device and corresponds to a forward direction of the shoe sole in the sole plane, when the measuring device 110 is correctly inserted into the shoe sole. Signal 502 is a derived signal that indicates a signal peak for the last portion of the positive acceleration spike of each step cycle represented in curve 506. The peak directly transitions into the plateau phase represented by signal 504, which corresponds to the time period where the foot is in contact with the supporting surface. This phase previously was denoted with the terms stance, contact or support phase. Derived signals 508, 510 and 512 each show respective step lengths determined for the y, x and z directions. In the shown example, the movement of the player is highly homogeneous, which is reflected in the similarity of the acceleration signal component *ay* for each step cycle and the identical values of the step length illustrated for each cycle period. It is to be noted that the shown step lengths are not measured contemporarily with the acceleration component signal *ay* shown in curve 506, but are determined from the portion of the curve 506 corresponding to each cycle and only displayed in parallel to the acceleration curve for illustration purposes.

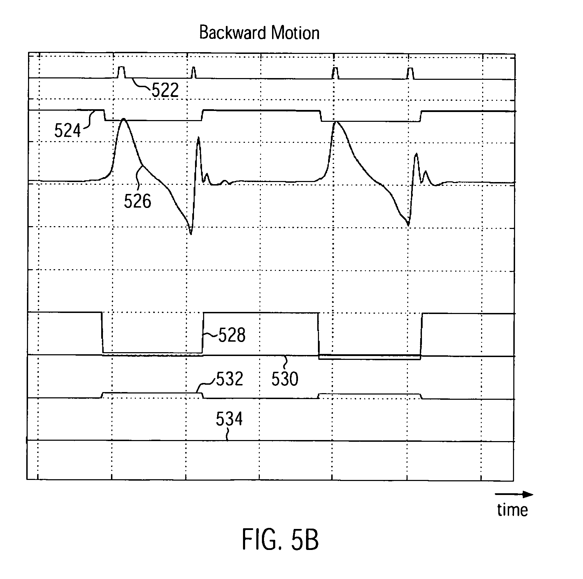

Referring now to Figure 5B, which corresponds to a backward motion, it is apparent that the negative step length in the y-direction represented with step length signal 528 differs in height, meaning that the derived step length, which is determined on the basis of the type of locomotion, the swing time and the characterizing acceleration values determined for each swing phase, varies for the different step cycles.

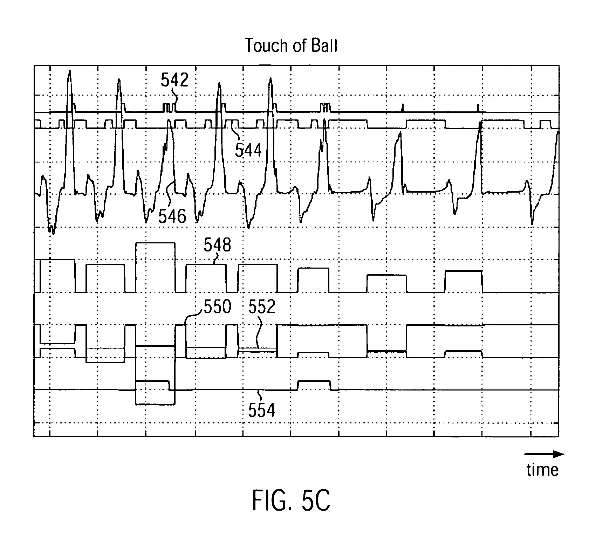

Signals 514, 534 and 554 in Figures 5A, 5B and 5C, respectively, show a ball contact signal. While during the forward motion and the backward motion illustrated in Figures 5A and 5B, no ball detection signals have been received by the measuring device 110, signal 554 in Figure 5C shows two ball detection events, which coincide with respective peak patterns in signal 542, thus indicating that the measuring device can register the touch event as a verified ball contact event of the monitored foot as described above.

Referring again to Figures 5A and 5B, it is apparent that the temporal sequence of minimum, maximum and upwards and downwards zero-crossing accelerations is drastically different between curves 506 and 526, representing a forward and a backward motion, respectively. In particular, curve 506 representing a forward motion shows the following sequence of states associated with the measured acceleration component:

1. plateau
2. plateau end
3. local maximum of *ay* (in a higher resolution applied for the state evaluation of the curve, this first local maximum is followed by a local minimum and another local maximum)
4. downward zero-crossing
5. absolute minimum of *ay*
6. upward zero-crossing
7. absolute maximum of *ay*, peak of *ay*
8. return to zero, start of plateau
9. plateau

In comparison, the temporal sequence of states derived from curve 526 representing the acceleration component *ay* for the backward motion as depicted in Fig. 5B is as follows:

1. plateau
2. plateau end
3. absolute maximum of *ay*
4. downward zero-crossing
5. absolute minimum of *ay*
6. upward zero-crossing
7. local maximum of *ay*
8. return to zero, start of plateau
9. plateau

According to the present invention, the type of locomotion, either a forward or a backward motion, in the example described as illustrated in Figures 5A and 5B, can be determined on the basis of the temporal sequence of the measured acceleration values. According to a preferred embodiment, this is accomplished by applying a state machine that employs a finite number of discrete states and transitions between these states according to respectively defined actions.

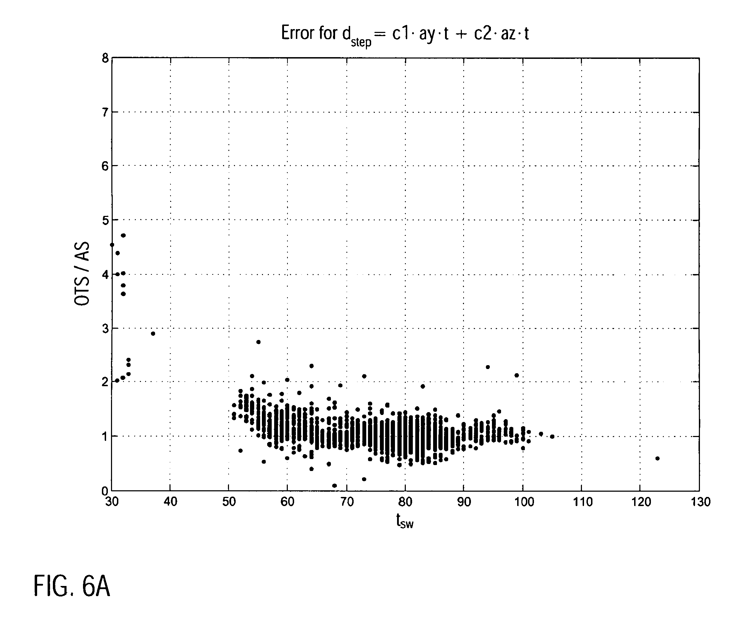

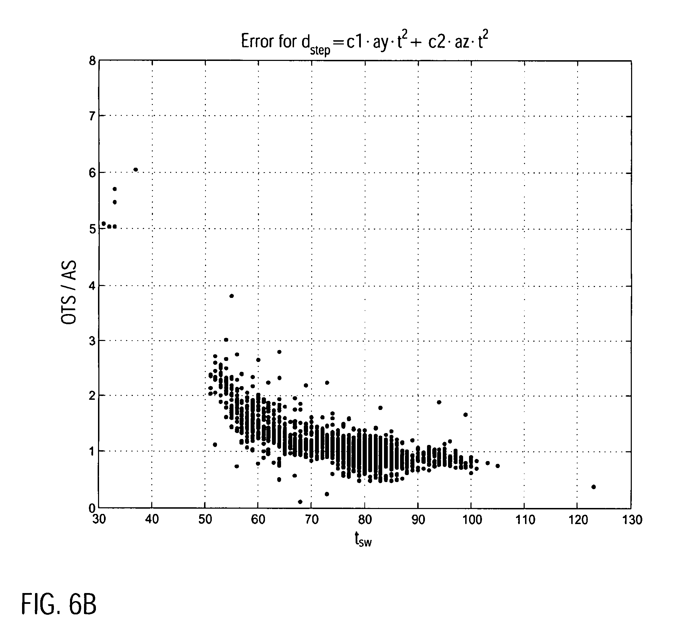

Referring now to Figures 6A and 6B, relative errors for determined step lengths $d_{step}$ are shown for exemplary calculation instructions in dependence of a swing time regime depicted on the x-axis and denoted with $t_{SW}$. The relative error is determined by calculating a quotient of a step length measured with a high-precision optical tracking system and a step length $d_{step}$ as measured by a device and method according to the present invention. The calculation instructions for $d_{step}$ differ in Figures 6A and 6B, wherein $d_{step}$ according to Figure 6A is calculated by equation (2):

$$(2) \quad d_{step} = c1 \cdot ay \cdot t + c2 \cdot az \cdot t \, ,$$

while $d_{step}$ according to Figure 6B is calculated by equation (3)

$$(3) \quad d_{step} = c1 \cdot ay \cdot t^2 + c2 \cdot az \cdot t^2 .$$

It is to be noted that parameters c1 and c2 after comparison with the step length measured by the optical tracking system may be calibrated in a manner resulting in a quotient of the two differently derived step lengths being close to a constant "1". Respectively fine-calibrated parameters are denoted with C1 and C2. As apparent from Figures 6A and 6B, the initial relative error for the underlying type of locomotion is smaller for calculation instructions according to equation (2). However, there may be locomotion regimes where equation (3), in particular after fine-calibration, may afford results of a higher precision.

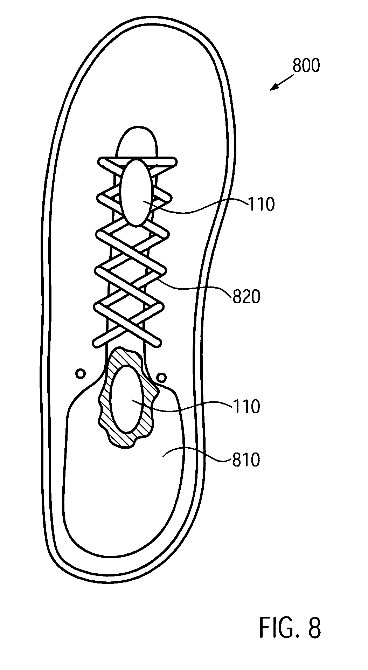

Referring finally to Figure 8, placement of a measuring device 110 either including an extended communication means and improved storing means to thus obviate a separate receiver device 120, or in communication with such a separate receiver device 120, is shown. The foot pod measuring device 110 is attached to a sports shoe 800 either clipped beneath laces 820 or inserted into a pocket provided for the foot pod in the shoe sole 810, preferably from the inside of the shoe after removing one or more insoles of the shoe.

## Claims

1. A device-implemented method of measuring movement of a person, the method comprising the steps of:

    continuously measuring (410) acceleration values associated with the locomotion of a person;
    determining (420), for a step of the person, a type of locomotion by analyzing a temporal sequence of acceleration values of a portion of the acceleration values corresponding to the step;
    determining (430), for the step, a swing time and at least one characterizing acceleration value from the acceleration values; and
    determining (440) a step length based on the type of locomotion, the swing time and the at least one characterizing acceleration value.

2. The method according to claim 1, further comprising the step of:

    calculating (460) a velocity associated with the locomotion of the person based on the step length.

3. The method according to claim 1 or 2, further comprising:

    determining a step length for each portion of the acceleration values; and
    calculating (470) the distance covered by the person based on of the determined step lengths.

4. The method according to one of claims 1 to 3, further comprising the step of:

    storing (405) a plurality of parameter sets, each set assigned to a type of locomotion,
    wherein the determining a step length comprises applying a parameter set assigned to the determined type of locomotion.

5. The method according to claim 4, wherein one or more of the plurality of parameter sets further correspond to at least one of:

    a velocity range;
    a characteristic of acceleration values corresponding to a sequence of steps; and
    data corresponding to a person, comprising physical properties of the person including age, weight and fitness level, and/or individually input selection parameters.

6. The method according to one of claims 4 to 5, further comprising the step of:

    calibrating (450) a parameter set by comparing step lengths, derived distances or velocities, which are determined based on the parameter set, with step lengths, distances or velocities determined with an optical tracking system, and applying the results of the comparison to the parameter set.

**7.** The method according to one of claims 1 to 6, wherein:

the acceleration values are multi-dimensional acceleration values sensed by at least one acceleration sensor (210);
the analyzing a temporal sequence of acceleration values comprises analyzing a first one-dimensional component of the multi-dimensional acceleration values; and
the at least one characterizing acceleration value comprises characterizing acceleration values for at least two one-dimensional components of the multi-dimensional acceleration values comprising the first one-dimensional component.

**8.** The method according to one of claims 1 to 7, wherein the at least one characterizing acceleration value determined for a step is based on a maximum, minimum, mean or weighted mean of corresponding acceleration values.

**9.** The method according to one of claims 1 to 8, wherein the acceleration values are measured with at least one acceleration sensor attached to one or more limbs (310, ..., 340) of the person (300).

**10.** A measuring device (110) for measuring movement of a person (300), the device comprising:

at least one acceleration sensor (210) for continuously measuring acceleration values associated with the locomotion of a person in at least two dimensions;
a storing means (220) for storing a plurality of parameter sets, each set assigned to a type of locomotion; and
a processing means (230) for assigning a type of locomotion to a step of the person by analyzing a temporal sequence of acceleration values of a portion of the acceleration values corresponding to the step; determining, for the step, a swing time and at least one characterizing acceleration value from the acceleration values; and determining a step length based on the swing time, the at least one characterizing acceleration value and the type of locomotion.

**11.** The measuring device according to claim 10, wherein
the processing means is further adapted to calculate a velocity associated with the locomotion of the person from the step length and/or to determine a step length for each portion of the acceleration values; and/or to calculate the distance covered by the person based on the determined step lengths.

**12.** The measuring device according to claim 10 or 11, further comprising
a communication means (240) for exchanging data with at least one of a receiver device (120), a game ball (150) and a computing device (140).

**13.** The measuring device according to one of claims 10 to 12, wherein the measuring device is a foot pod adapted for attachment to a sports shoe or insertion into a shoe sole.

**14.** A system (100) comprising the measuring device (110) according to one of claims 10 to 13, further comprising
a receiver device (120) for receiving data from the measuring device, storing the data, displaying a graphical representation of the data and/or transmitting sets of the stored data to a computing device (140).

**15.** A system according to claim 14, further comprising
a game ball (150) adapted to send touch detection signals to the measuring device (110).

Game Ball
150

100

Measuring Device 110 ⟷ Receiver Device 120

Display 130

Computing System 140

FIG. 1

110

Power Supply 250

MFS 270

Storing Means 220

Acceleration Sensor(s) 210

Processing Means 230

Communication Means 240

FIG. 2

**FIG. 3**

400

Start —401

Store Parameter Sets —405

Calibrate Parameter Sets —408

Measure Acceleration —410

Type of locomotion —420

Determine $T_{sw}$, $a_y$, $a_z$, ... —430

Determine $d_{step.}$ —440

Calibrate Parameter Set —450

Calculate v —460

Calculate distance —470

End —499

**FIG. 4**

Forward Motion

FIG. 5A

Backward Motion

time

FIG. 5B

Touch of Ball

FIG. 5C

Error for $d_{step} = c1 \cdot ay \cdot t + c2 \cdot az \cdot t$

FIG. 6A

Error for $d_{step} = c1 \cdot ay \cdot t^2 + c2 \cdot az \cdot t^2$

FIG. 6B

EP 2 284 493 A1

Test: Different speed profiles on 50 meter track for 10minutes

FIG. 7A

FIG. 7B

FIG. 8

Europäisches Patentamt
European Patent Office
Office européen des brevets

**EUROPEAN SEARCH REPORT**

Application Number

EP 09 01 0312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 145 389 A (EBELING W H CARL [US] ET AL) 14 November 2000 (2000-11-14) | 1-5,7-14 | INV.<br>G01C22/00 |
| Y | * the whole document * | 6 | A61B5/103 |
| A | | 15 | A63B69/00<br>G01P1/12 |
| | ----- | | |
| X | US 2007/208544 A1 (KULACH CHRISTOPHER J [CA] ET AL) 6 September 2007 (2007-09-06) | 1-5,7-15 | |
| A | * paragraphs [0025], [0030], [0043] - [0058], [0079], [0086] * | 6 | |
| | ----- | | |
| X | US 2008/214360 A1 (STIRLING ROSS G [CA] ET AL) 4 September 2008 (2008-09-04) * paragraphs [0041], [0075], [0082] - [0086], [0105], [0117], [0118], [0122] * | 1-5,7-14 | |
| | ----- | | |
| X | US 2004/064286 A1 (LEVI ROBERT W [US] ET AL) 1 April 2004 (2004-04-01) * paragraphs [0006], [0018], [0024] - [0029], [0034], [0038], [0041] * | 1,10 | |
| | ----- | | TECHNICAL FIELDS SEARCHED (IPC) |
| X | US 2008/255800 A1 (MERIHEINA ULF [FI] ET AL) 16 October 2008 (2008-10-16) * the whole document * | 1,10 | G01C<br>A61B<br>A63B<br>G01P |
| | ----- | | |
| Y | BE 1 014 748 A6 (LANGE PHILIPPE [BE]; HOEST PIERRE L [BE]) 2 March 2004 (2004-03-02) * figure 8 * | 6 | |
| | ----- | | |
| A | EP 1 847 807 A1 (HONEYWELL INT INC [US]) 24 October 2007 (2007-10-24) * paragraphs [0030] - [0033], [0061], [0062] * | 1,10 | |
| | ----- | | |
| A | US 6 356 856 B1 (DAMEN ERIK P N [NL] ET AL) 12 March 2002 (2002-03-12) * the whole document * | 1,10 | |
| | ----- | | |

The present search report has been drawn up for all claims

| Place of search | Date of completion of the search | Examiner |
|---|---|---|
| The Hague | 7 January 2010 | Bruinsma, Maarten |

EPO FORM 1503 03.82 (P04C01)

## ANNEX TO THE EUROPEAN SEARCH REPORT
## ON EUROPEAN PATENT APPLICATION NO.

EP 09 01 0312

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2010

| Patent document cited in search report | | Publication date | Patent family member(s) | | Publication date |
|---|---|---|---|---|---|
| US 6145389 | A | 14-11-2000 | NONE | | |
| US 2007208544 | A1 | 06-09-2007 | EP<br>WO<br>US | 1991877 A2<br>2008010097 A2<br>2009076765 A1 | 19-11-2008<br>24-01-2008<br>19-03-2009 |
| US 2008214360 | A1 | 04-09-2008 | NONE | | |
| US 2004064286 | A1 | 01-04-2004 | NONE | | |
| US 2008255800 | A1 | 16-10-2008 | CA<br>EP<br>WO | 2615211 A1<br>2137493 A1<br>2008125730 A1 | 13-10-2008<br>30-12-2009<br>23-10-2008 |
| BE 1014748 | A6 | 02-03-2004 | NONE | | |
| EP 1847807 | A1 | 24-10-2007 | US | 2007250261 A1 | 25-10-2007 |
| US 6356856 | B1 | 12-03-2002 | CN<br>DE<br>DE<br>ES<br>WO<br>JP<br>JP | 1256752 A<br>69921040 D1<br>69921040 T2<br>2230831 T3<br>9944016 A1<br>4286328 B2<br>2002500768 T | 14-06-2000<br>18-11-2004<br>09-03-2006<br>01-05-2005<br>02-09-1999<br>24-06-2009<br>08-01-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 6513381 B2

• CA 2615211 A1